# EUROPÄISCHE PATENTSCHRIFT

(11) **EP 0 669 342 B1**
(45) Veröffentlichungstag und Bekanntmachung des Hinweises auf die Patenterteilung: **05.09.2001**
(21) Anmeldenummer: 95102504.8
(22) Anmeldetag: 22.02.1995
(51) Int. Cl.: C07K 1/16, C07K 14/745, C12N 9/64

(54) **Verfahren zur Isolierung und Reinigung Vitamin K-abhängiger Proteine**
Process to isolate and purify vitamin-K dependent proteins
Procédé d'isolation et de purification des protéines vitamine-K dépendents

(30) Priorität: 28.02.1994 DE 4406515
(43) Veröffentlichungstag der Anmeldung: 30.08.1995
(73) Patentinhaber: Baxter Aktiengesellschaft, 1221 Wien (AT)
(72) Erfinder: Fischer, Bernhard, Dr., A-1120 Wien (AT); Mitterer, Artur, Dr., A-2304 Orth/Donau (AT); Dorner, Friedrich, Prof., A-1230 Wien (AT)
(74) Vertreter: Kolb, Helga, Dr. Dipl.-Chem.

(56) Entgegenhaltungen:
- EP-A- 0 056 629
- EP-A- 0 317 376
- EP-A- 0 363 126
- 9TH INTERNATIONAL SYMPOSIUM ON AFFINITY CHROMATOGRAPHY AND BIOLOGICAL RECOGNITION, YOKOHAMA, JAPAN, SEPTEMBER 24-28, 1991. J CHROMATOGR 597 (1-2). 1992. 285-291. CODEN: JOCRAM ISSN: 0021-9673, XP002024897 OHSAWA K ET AL: "PURIFICATION OF SUFFICIENTLY GAMMA-CARBOXYLATED RECOMBINANT PROTEIN C AND ITS DERIVATIVES CALCIUM -DEPENDENT AFFINITY SHIFT IN IMMUNOAFFINITY AND ION - EXCHANGE CHROMATOGRAPHY."
- JOURNAL OF BIOTECHNOLOGY 38 (2). 1995. 129-136. ISSN: 0168-1656, XP002024898 FISCHER B ET AL: "Purification of recombinant human coagulation factors II and IX and protein S expressed in recombinant Vaccinia virus-infected Vero cells."

## Beschreibung

Neben den natürlich vorkommenden Proteinen ist es heute mit Hilfe gentechnologischer Methoden möglich, Säugerproteine, insbesondere menschliche Proteine, rekombinant herzustellen. Dazu werden mit Fremd-DNA transformierte oder transfizierte Wirtszellen kultiviert, wobei im Falle eukaryontischer Wirtszellen das rekombinant hergestellte Protein in löslicher Form in das Zellkulturmedium abgegeben wird (R.G. Werner und W. Berthold, Drug Res. 38, 422-428 (1988)). Da die Wirtszellen neben dem gewünschten, rekombinant hergestellten Protein aber auch noch andere Proteine in das Zellkulturmedium abgeben, ist es erforderlich, das gewünschte Protein in einem oder mehreren Reinigungsschritten anzureichern bzw. zu isolieren. Hierzu bedarf es Verfahren, die effektiv und selektiv die Isolierung rekombinant hergestellter Proteine aus dem Zellkulturmedium erlauben.

Für die Reinigung rekombinanter Proteine nutzt man in der Regel die physikalischen und chemischen Eigenschaften der Proteine. Solche Eigenschaften sind z.B. die Grösse der Proteine, die natürliche Ladung der Oberfläche, deren Hydrophilie oder Löslichkeit. Weitere Reinigungsverfahren beruhen auf der Bindung mit anderen Molekülen, wie z.B. Antikörpern. Ähnliches gilt für die Reinigung von Proteinen aus natürlichen Quellen. Auch hier erfolgt aufgrund der physikalischen und chemischen Eigenschaften eines Proteins eine Abtrennung desselben von den übrigen Begleitproteinen.

Auch Vitamin K-abhängige Proteine wurden bisher nach solchen Methoden, wie z.B. Proteinfällung, Ionenaustausch-Chromatografie und Gelfiltration gereinigt (A.L. Bloom und D.P. Thomas, Haemostasis and Thrombosis, Churchill Livingstone, New York, 1987). B. Dahlbäck (Biochem. J. 209, 837-846 (1983)) beschreibt ein Reinigungsverfahren für Protein S (PS). Nach einer Bariumcitrat-Fällung wird PS durch Chromatographie an DEAE-Sephacel gewonnen. J. Malm et al (Eur. J. Biochem. 187, 737-743 (1990)) reinigten rekombinantes PS (rPS) durch Affinitätschromatographie an einem monoklonalen Antikörper. Faktor IX (FaIX) wurde durch B. Osterude und R Flengsrud (Biochem. J. 145, 469-474 (1975)) durch Bariumsulfat-Fällung und Ionen-Austauschchromatograpie an Cellulose isoliert. Monoklonale Antikörper wurden durch H. Kim et al (Sem. Hematol. 28 Suppl. 6, 15-19 (1991)) zur Reinigung von FaIX benutzt.

US-5,055,557 beschreibt ein Verfahren zur Reinigung und Konzentrierung Vitamin K-abhängiger Proteine aus Plasma sowie auch rekombinant hergestellter Proteine mit Hilfe eines Immunadsorbens unter Verwendung eines monoklonalen Antikörper.

Bei den vorstehend genannten Verfahren ist zu beachten, dass sich die Eigenschaften vieler Proteine nicht wesentlich voneinander unterscheiden, was ihre Auftrennung erschwert. Es ist daher notwendig, Kombinationen von fein aufeinander abgestimmten Reinigungsschritten anzuwenden, um Unterschiede in den Proteineigenschaften optimal auszunutzen.

Eine Reihe von Proteinen, die natürlicherweise in menschlichem und tierischem Blut vorkommen, können zweiwertige Kationen binden. Solche Proteine werden in einem Vitamin K-abhängigen Prozess in den Zellen synthetisiert, wobei durch die Umwandlung von Glutaminsäure (Glu) in gamma-Carboxy-glutaminsäure (Gla) Kationenbindungsstellen entstehen. Diese Kationenbindungsstellen können dann abgesättigt werden durch Calicumionen (Ca²⁺). Ausser Calciumionen vermögen diese Kationenbindungsstellen in der Regel auch zweiwertiges Strontium oder Barium zu binden (B. Furie und B.C. Furie, Cell 53, 508-518 (1988).

Viele dieser Vitamin K-abhängigen Proteine sind Bestandteil des Blutplasmas und haben eine wichtige Bedeutung bei der Haemostase. In diesen Vitamin K-abhängigen Proteinen befinden sich die gamma-Carboxy-glutaminsäurereste in den strukturell homologen N-terminalen Gla-Regionen (A. Tulinsky, Thromb. Haemost. 66, 16-31 (1991)). Zu diesen Calciumionen bindenden Proteinen mit homologer Gla-Region gehören u.a. Protein S (PS), Protein C, Faktor IX (FaIX), Faktor II, Faktor VII, Faktor X und Protein Z.

Infolge der Bindung von Calciumionen zeigen diese Proteine deutlich veränderte Eigenschaften im Vergleich zu nicht-Calcium-bindenden Proteinen. Dieser Unterschied wird in EP-363 126 zur Reinigung von Proteinen ausgenutzt. EP-363 126 beschreibt ein Verfahren zur Isolierung von Vitamin K-abhängigen Proteinen, die rekombinant gewonnen wurden. Dabei werden durch Zugabe eines Chelatbildners zum Kulturmedium vor der eigentlichen Proteinisolierung die divalenten Kationen vollständig entfernt und das Protein dadurch befähigt, an ein Ionenaustauscherharz, wie Mono Q, zu binden. Daraufhin wird durch Zusatz von NaCl und Ca²⁺-Ionen das Protein vom Anionenaustauscher eluiert. Dabei wurde ein 58% angereichertes Produkt (Protein C) erhalten. Die Verunreinigungen (42%) stellen solche Proteine dar, die bei der angewendeten Salzkonzentration (0,15M) ebenfalls vom Ionenaustauscher gelöst werden. In einem weiteren Reinigungsschritt wird der erhaltene Protein-Kation-Komplex an eine Säule, an die immobilisiertes EDTA gebunden ist, adsorbiert, gewaschen und das Protein eluiert. Die Proteine im Eluat werden an einen nachgeschalteten Ionenaustauscher gebunden und mit einem Salzgradienten eluiert. Nach Zugabe von CaCl₂ ins Eluat wird der Protein-Kation-Komplex an eine hydrophobe Säule adsorbiert und das gewünschte Protein durch EDTA-Puffer eluiert.

Nachteil des in EP-363 126 beschriebenen Verfahrens ist, dass durch mehrfaches Entfernen und Zugeben von Calciumionen eine häufige Konformationsänderung des Vitamin K-abhängigen Proteins erfolgt, was zu Veränderungen der Proteineigenschaften führt (Johanson et al, J. Biol. Chem. 258, 5554-5560 (1983); J Stenflo, J. Biol. Chem. 251, 355-363 (1976)). Demgegenüber wird mit dem erfindungsgemässen Verfahren eine Methode zur Verfügung gestellt, die diese Nachteile nicht aufweist, da während des gesamten Reinigungsprozesses kein Verfahrensschritt zur Entfernung von zweiwertigen Metallionen z.B. durch Chelatbildner notwendig ist.

Vorteil der vorliegenden Erfindung ist daher, dass die ursprüngliche Konformation und Stabilität des Vitamin K-abhängigen Proteins durch die Anwesenheit des CaCl₂ während der Reinigung erhalten bleibt.
EP-354 354 beschreibt ein Verfahren zur Anreicherung der Blutgerinnungsfaktoren II, VII, IX und X. Die vorstehend genannten Prothrombin-Koinplexfaktoren werden durch Adsorption an einer alpha-Hydroxyl-Aminogruppen-tragenden Matrix gewonnen, wobei insbesondere Faktor IX stark gebunden wird. In Abhängigkeit von der Ionenstärke werden die Faktoren II, VII und X vorher eluiert. Die Zugabe von Calciumionen spielt bei diesem Verfahren keine Rolle.

Ohsawa, K. et al. (*Journal of Chromatography*, **597**, (1992), 285-291) beschreibt die Trennung von ausreichend γ-carboxyliertem Protein C von ungenügend carboxylierten Protein C durch Affinitätschromatographie und Dialyse der Lösung gegen Tris-HCl Puffer enthaltend CaCl2. Anschließend wird die Probe auf eine DEAE-Säule aufgetragen und mit linearen Gradienten eluiert. Das gereinigte Protein C wird an die Säule gebunden und danach durch steigende NaCl Konzentration eluiert. In Gegenteil zu D2 in vorliegender Anmeldung werden ausgehend von einer ungereinigten Lösung nur Begleitproteine an das Anionenaustauschmaterial adsorbiert, und nicht Vitamin K-abhänglge Proteine.

EP-317 376 beschreibt ein Verfahren zur Abtrennung einer Faktor IX-enthaltenden menschlichen Plasmafraktion, wobei Kryopräzipitat zunächst chromatografisch vorgereinigt, dann eine Trennung durch Anionenaustauschchromatographie und selektive Elution durch einen Puffer mit zunehmender Ionenstärke vorgenommen und schliesslich eine Affinitätschromatographie an Heparin-Sepharose mit selektiver Elution durchgeführt wird. Auch dieses Verfahren berücksichtigt nicht die veränderten Proteineigenschaften von Faktor IX in Gegenwart oder Abwesenheit von Calciumionen.

Aufgabe der vorliegenden Erfindung ist es, ein Verfahren zur Trennung Vitamin K-abhängiger und Calciumionen-bindender Proteine von nicht-Vitamin K-abhängigen Begleitproteinen aus Lösungen zur Verfügung zu stellen, ohne dass die Notwendigkeit des Entfernens von Calciumionen und eine damit möglicherweise verbundene Denaturierung der Proteine erforderlich ist. Dieses Verfahren, welches man mit angereicherten Proteinlösungen aus natürlichen Quellen sowie mit Zellkulturüberständen der rekombinanten Proteinherstellung durchführen kann, soll einfach sein und zu hochreinen Vitamin K-abhängigen Proteinen führen.

Die vorstehend Aufgabe wird gemäss der Erfindung durch die in den Ansprüchen 1 bis 16 aufgezeigten Verfahren gelöst. Die Erfindung umfasst auch die mit Hilfe dieser Verfahren gewonnenen hochreinen, Vitamin K-abhängigen Proteine.

Bei dem erfindungsgemässen Verfahren werden z.B. Zellkulturüberstände sowie anderweitig aufgearbeitete Zellkulturmedien, Überstände von Gewebezellkulturen oder Proteinlösungen aus natürlichen Quellen, die Vitamin K-abhängige Proteine enthalten, zunächst über einen Anionenaustauscher chromatographiert. Ein spezifisches Entfernen von divalenten Kationen aus der Proteinlösung vor dem Chromatographieschritt ist im Gegensatz zu dem in EP-363 126 beschriebenen Verfahren nicht erforderlich. Vielmehr wurde überraschenderweise festgestellt, dass durch Zugabe von geringen Mengen an Calciumionen bei geringer Salzstärke keine Bindung des Vitamin K-abhängigen Proteins, jedoch von nicht-Vitamin K-abhängigen Proteinen, an den Anionenaustauscher erfolgt. Bei dem erfindungsgemässen Verfahren zur Trennung von Vitamin K-abhängigen und nicht-Vitamin K-abhängigen Proteinen wurde erstmals ein neuartiger Anionenaustauscher mit Hyperdiffusionseigenschaften, z.B. Q Hyper D® (Sepracor) verwendet. An diese Anionenaustauschchromatographie schliesst sich dann - sofern eine weitere Proteinreinigung gewünscht wird - eine Affinitätschromatographie an.

Erfindungsgemäss kann die Vitamin K-haltige Proteinlösung, durch einen zusätzlichen Proteinkonzentrationsschritt ohne vorheriges Entfernen von divalenten Kationen aus der Proteinlösung angereichert werden, anschliessend durch einen Anionenaustauscher filtriert, sowie gegebenenfalls über eine Affinitätssäule chromatographiert werden.

Nach einer bevorzugten Alternative wird das in einem Vorreinigungsschritt (1. Schritt) angereicherte Proteinkonzentrat durch einen Anionenaustauscher (2. Schritt) filtriert und dann direkt auf eine Affinitätssäule aufgetragen. Diese bevorzugte Kombination ist wie folgt gekennzeichnet:
(a) Eine Lösung, die ein Vitamin K-abhängiges Protein enthält, wird mit Anionenaustauscher in Kontakt gebracht, wobei das Vitamin K-abhängige Protein an den Austauscher adsorbiert und anschliessend bei zunehmender Salzkonzentration eluiert wird; daraufhin wird die Salzkonzentration des Eluats reduziert;
(b) dem Eluat aus (a) werden Calciumionen zugegeben und dieses wird wiederum unter Bedingungen mit Anionenaustauscher in Kontakt gebracht, bei denen die Begleitproteine, jedoch nicht das Vitamin K-abhängige Protein adsorbiert werden;
(c) das nicht-adsorbierte Vitamin K-abhängige Protein von (b) wird an einen Affinitätsträger adsorbiert und selektiv eluiert.

Die vorstehend genannte, bevorzugte Kombination von Verfahrensschritten wird nachfolgend noch näher erläutert:

Für den Proteinkonzentrierungsschritt (1. Schritt) bringt man die Lösung, welche ein rekombinantes oder natürliches Vitamin K-abhängiges Protein enthält, in Kontakt mit einem Anionenaustauscher. Dies kann durch einfaches Vermischen oder durch Leiten über eine Säule erfolgen. Dabei befindet sich das Vitamin K-abhängige Protein zusammen mit den Begleitproteinen in einer Salzlösung niedriger Konzentration (Minimalsalzkonzentration). Unter diesen Bedingungen wird das Vitamin K-abhängige Protein zusammen mit einer Reihe anderer Proteine an den Anionenaustauscher gebunden. Durch Erhöhung der Salzkonzentration (Ionenstärke) wird dann im folgenden das Vitamin K-abhängige Protein vom Austauscher selektiv abgelöst.

Zellkulturüberstände enthalten in der Regel einen Farbstoff, der den pH-Status anzeigt. Dieser Farbstoff, wie z.B. Phenolrot, trübt die klare Proteinlösung, bindet stark an übliche Anionenaustauscher, wie Q Sepharose Fast Flow (Pharmacia) oder MacroPrep® (Bio-Rad) und absorbiert das Licht bei der Wellenlänge der Proteinbestimmung, i.e. 280 nm. Dies führt zu einer Erschwerung der Reinigung von Proteinen aus Zellkulturmedien. Um hier eine Verbesserung zu schaffen, wurden gemäss der Erfindung Zellkulturüberstände erstmals über einen Anionenaustauscher mit Hyperdiffusionseigenschaften (Q-Hyper D ®, Sepracor) filtriert. Dabei zeigte sich, dass bei Verwendung von Q-Hyper D als Anionenaustauscher die unspezifische Bindung des Farbstoffes Phenolrot auf ein Minimum reduziert ist. Der Farbstoff wird bereits bei Salzkonzentrationen unterhalb der Minimalkonzentration vom Anionenaustauscher eluiert. Auf diese Weise konnten während der Chromatographie die eluierten Proteine besser nachgewiesen werden. Ausserdem wird eine störende Konkurrenzreaktion zwischen den Proteinen und dem Farbstoff am Ionenaustauscher weitgehend vermieden.

Die weitere Anionenaustauschchromatographie (2.Schritt) besteht darin, dass durch Dialyse oder Verdünnen mit salzfreiem Puffer die Ionenstärke der das Vitamin K-abhängige Protein enthaltenden Lösung auf einen Wert unterhalb der Minimalsalzkonzentration reduziert und geringe Mengen an Calciumionen zugegeben werden. Die Proteinlösung wird dann noch einmal mit dem Anionenaustauscher in Kontakt gebracht bzw. durch diesen filtriert. Dabei wurde überraschenderweise festgestellt, dass Vitamin K-abhängige Proteine nicht an den Anionenaustauscher binden und die nicht-Vitamin K-abhängigen Begleitproteine an den Träger adsorbieren. Dies ist eine Folge der unterschiedlichen Ladung des Proteins infolge der Bindung von Ca-Ionen. Das von den kontaminierenden Proteinen der Vorstufe nunmehr weiter gereinigte Vitamin K-abhängige Protein wird dabei direkt (ohne weitere salzabhängige Elution) erhalten.

In vielen Fällen der Proteinreinigung ist eine Kombination einer Vorreingungsstufe (1.Schritt) mit einer Reinigung am Anionenaustauscher (2.Schritt) ausreichend. Dabei ist der zweite Reinigungsschritt umso effektiver, je weniger das abzutrennende, Vitamin K-abhängige Protein mit Begleitproteinen kontaminiert ist.

Im zweiten Reinigungsschritt am Anionenaustauscher wird durch die Wahl einer bestimmten Salzkonzentration unterhalb der Minimalsalzkonzentration der Bandbreite und durch die Zugabe von Calciumionen das Vitamin K-abhängige Protein nicht am Austauscher gebunden.

Durch die Ausnutzung der Calcium-Bindungseigenschaften der Vitamin K-abhängigen Proteine mit Gla-Region werden diese dadurch von Begleitproteinen getrennt, dass bei bestimmten Ionenstärken nur die Begleitproteine an den Anionenaustauscher gebunden werden, jedoch die Vitamin K-abhängigen Proteine den Anionenaustauscher, ohne an diesen zu binden, passieren. Das erfindungsgemässe Verfahren eignet sich daher neben Vitamin K-abhängigen Proteinen aus natürlichen Quellen in besonderem Masse zur Reinigung von rekombinanten, Vitamin K-abhängigen Proteinen mit Gla-Region, wie z.B. Protein C, Faktor IX, Faktor II, Faktor VII, Protein S, Protein Z und Faktor X.

Es ist bevorzugt, für die vorstehend genannten Schritte 1 und 2 den gleichen Anionenaustauscher zu verwenden, wobei besonders gute Ergebnisse erhalten werden, wenn die AnionenaustauschChromatographie auf Säulen erfolgt.

In dem nachgeschalteten Verfahren (3.Schritt), nämlich der Affinitätschromatographie, erfolgt eine Bindung des Vitamin K-abhängigen Proteins an den Affinitätsträger. Bei diesem Schritt wird das Vitamin K-abhängige Protein an den Affinitätsträger adsorbiert. Hierbei erweist sich die vorgeschaltete Vorreinigungsstufe am Anionenaustauscher als besonders vorteilhaft, da durch diese bereits eine Reihe von kontaminierenden Proteinen entfernt wurde. Dies begünstigt die Elution des Vitamin K-abhängigen Proteins vom Affinitätsträger und erlaubt die Gewinnung des gewünschten Proteins in hoher Reinheit.

Das erfindungsgemässe Verfahren kann einen dem Fachmann aus dem Stand der Technik bekannten Virusinaktivierungsschritt beinhalten, der die Behandlung der Vitamin K-abhängigen Proteinlösungen mit physikalisch-chemischen oder chemischen Methoden umfasst. Dazu zählt z.B. die Behandlung in Gegenwart von viruziden Substanzen, gegebenenfalls kombiniert mit einer Strahlen- oder Wärmebehandlung. Gemäss der vorliegenden Erfindung kann die Virusinaktivierung vor der Trennung von Vitamin K-abhängigen von nicht-Vitamin K-abhängigen Begleitproteinen erfolgen.

Das erfindungsgemässe Verfahren kann sich aus einer Kombination der Reinigungsschritte 1, 2 und 3 zusammensetzen, die in beliebiger Reihenfolge durchgeführt werden können. Obligatorisch ist dabei jedoch immer der Verfahrensschritt gemäss Anspruch 1.

Die Figuren 1 bis 12 zeigen folgende Reinigungsschritte bzw. -ergebnisse:
- Fig.1: Reinigung von rekombinantem Protein S aus Zellkulturüberstand durch Chromatographie an Q-Hyper D (Vorstufe = 1. Schritt);
- Fig.2: Reinigung von rekombinantem Protein S durch Chromatographie an Q-Hyper D unter Zusatz von Calciumionen (= 2. Schritt);
- Fig. 3.: SDS-PAGE-Gelelektrophorese von gereinigtem rekombinantem Protein S (A: Zellkulturüberstand; B: gereinigtes rPS) ;
- Fig. 4: Reinigung von rFaIX durch Chromatographie an Q-Hyper D (1.Schritt)
- Fig. 5: Reinigung von rFaIX durch Chromatographie an Q-Hyper D unter Zusatz von Calciumionen (2.Schritt);
- Fig. 6: SDS-PAGE-Gelelektrophorese von rFa IX (A: Zellkulturüberstand; B: gereinigter rFa IX);
- Fig. 7: Reinigung von rFaIX durch Chromatographie an Heparin-Sepharose (3.Schritt);
- Fig. 8: Reinigung von rFaIX durch gekoppelte Chromatographie an Q-Hyper D und Heparin-Sepharose (2. und 3.Schritt);
- Fig. 9: SDS-PAGE-Gelelektrophorese von rFaIX, wie in Fig.8 erhalten.
- Fig.10: Reinigung von rFaII aus Zellkulturüberstand durch Chromatographie an Fractogel® EMD TMAE 650 (1.Schritt);
- Fig.11: Reinigung von rFaII durch Chromatographie an Fractogel® EMD TMAE 650 (2. Schritt);
- Fig.12: Analyse von rFaII durch SDS-PAGE-Gelelektrophorese (A: Zellkulturüberstand; B: gereinigter FaII).

Die folgenden Beispiele zeigen die erfindungsgemässe Reinigung von Protein S, Faktor IX und Faktor II.

### Beispiel 1

### a) Reinigung von rPS durch Anionen-Austausch-Chromatographie

Im folgenden Beispiel wurde ein quaternärer Amino-Typ Anionen-Austauscher mit Hyperdiffusionseigenschaften (Q-Hyper D, Sepracor) verwendet.

Materialien:
Säule: Q-Hyper D, Sepracor; 2 cm x 4 cm.
Puffer A: 20 mM Bis-Tris/HCl, pH 7,0.
Puffer B: 20 mM Bis-Tris/HCl, pH 7,0, 0,18 M NaCl.
Puffer C: 20 mM Bis-Tris/HCl, pH 7,0, 0,4 M NaCl.
Puffer D: 20 mM Bis-Tris/HCl, pH 7,0 1 mM NaCl.

Rekombinantes Protein S (rPS) wurde, basierend auf üblichen Laborverfahren, nach Infektion von Vero-Zellen (Affen-Nierenzellen) mit Vaccinia-Virus in Zellkulturtechnik gewonnen. Vero/Vaccinia Expressionssysteme und Zellkulturbedingungen sind in F.G. Falkner et al, Thrombosis and Haemostasis, 68, 119-124 (1992) und N Barrett et al, AIDS Res., 5, 159-171 (1989); F Dorner et al, AIDS Vaccine Research and Clinical Trials, Marcel Dekker, Inc. New York (1990), ausführlich beschrieben. Die Expression von rPS erfolgte in käuflich verfügbarem, synthetischem DMEM-Medium. Nach der Zellkultur wurde der Kulturüberstand durch Zentrifugation gewonnen und mit dem synthetischen Proteasehemmstoff Pefabloc® SC, Pentapharm, zu 0,1 mMol/l versetzt.

Die Säule wurde entsprechend der Vorschrift des Herstellers regeneriert und mit Puffer A equilibriert. Anschliessend wurden 485 ml Zellkulturüberstand, welcher rekombinantes Protein S enthielt, mit einer Geschwindigkeit von 10 ml/min auf die Säule aufgetragen. Das nicht an die Säule gebundene Material wurde bei gleicher Fliessgeschwindigkeit durch Waschen mit Puffer A entfernt. Daraufhin wurde die Säule zuerst mit Puffer B, anschliessend mit Puffer C eluiert. Die Nachelution erfolgte mit Puffer D. Während der Chromatographie wurde die Proteinadsorbtion in üblicher Weise bei 280 nm verfolgt. Nach Durchführung der Chromatographie wurde die Proteinkonzentration mittels der Bradford-Methode (M. Bradford, Anal. Biochem. 72, 248-254 (1976))bestimmt. Der Gehalt an Protein S wurde sowohl mittels eines handelsüblichen ELISA-Systems (Asserachrome Protein S, Boehringer Mannheim) als auch mittels eines Gerinnungstestes (Protein S Clotting-Test, Boehringer Mannheim) bestimmt.

Es wurde festgestellt, dass nahezu alles rPS an den Träger gebunden wurde. rPS wurde durch 0,4 M NaCl (Puffer C) vom Anionenaustauscher eluiert.

Bei Verwendung des vorstehend genannten Anionenaustauschers wird der üblicherweise im Zellkulturmedium enthaltene Farbstoff Bromphenolrot bereits bei einer Salzkonzentration von 0,18 M von der Säule eluiert, was die nachfolgende Isolierung von rPS bei 0,4 M NaCl wesentlich begünstigt. Dies stellt einen Vorteil gegenüber der Verwendung anderer Anionenaustauscher dar.

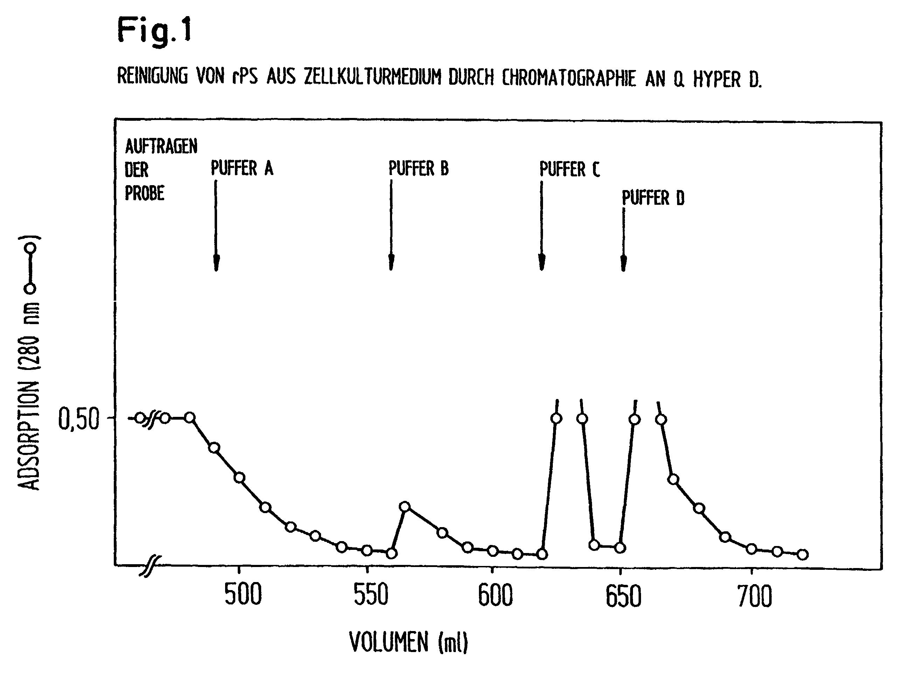

Die wesentlichen Ergebnisse der Reinigung von rPS am Anionenaustauscher (1. Schritt) sind in Fig. 1 und Tabelle 1 zusammengefasst. Durch die in Beispiel 1 beschriebene Reinigung wurde der Anteil von 3% rPS-Antigen zum Gesamtprotein des Zellkulturmediums auf 8% rPS-Antigen zum Protein der 0,4 M NaCl-Fraktion erhöht. Die spezifische Aktivität erhöhte sich um das 25-fache.

**Tabelle 1**

| Probe | Volumen | Protein | Protein S | | Spezif. Aktivität |
|---|---|---|---|---|---|
| | | | Antigen | Aktivatät | |
| | (ml) | (mg/ml) | (mU/ml) | (mU/ml) | (U/mg) |
| Zellüberstand | 485 | 0,108 | 137 | 11 | 0,1 |
| ungebundene Fraktion | 560 | 0,05 | 4 | 0 | 0 |
| 0,18 M NaCl | 60 | 0,014 | 4 | 0 | 0 |
| 0,4 M NaCl | 14 | 0,537 | 1700 | 1358 | 2,5 |
| 1 M NaCl | 20 | 0,54 | 4 | 0 | 0 |

### b) Reinigung von rPS durch Adsorption von Begleitproteinen mittels Anionen-Austausch-Chromatographie unter Zusatz von Calciumionen (2.Schritt)

Es wurde der gleiche Anionen-Austausch-Typ verwendet wie unter 1.a) beschrieben.

Materialien:
Säule: Q-Hyper D, Sepracor; 1 cm x 4 cm.
Instrument: Pharmacia FPLC LCC-500.
Puffer A. 20 mM Bis-Tris/HCl, pH 7,0.
Puffer B: 20 mM Bis-Tris/HCl, pH 7,0, 0,15 M NaCl, 10 mM CaCl₂.
Puffer C: 20 mM Bis-Tris/HCl, pH 7,0, 1 M NaCl.

Die Säule wurde entsprechend der Vorschrift des Herstellers regeneriert und mit Puffer B equilibriert. Rekombinantes Protein S, das wie in Beispiel 1.a) beschrieben aus Zellkulturüberstand isoliert worden war, wurde mit Puffer A 2,5-fach verdünnt, so dass die Konzentration von NaCl unter 0,18 M lag. Es wurde CaCl₂ mit: einer Konzentration von 10 mM zugegeben. Anschliessend wurde das Proteingemisch auf die Säule aufgetragen und mit Puffer B das ungebundene Protein aus der Säule gewaschen. Gebundenes Protein wurde mittels Puffer C eluiert. Der Verlauf der Chromatogaphie wurde wie in Beispiel 1.a) beschrieben verfolgt und die jeweilige Proteinkonzentration bestimmt.

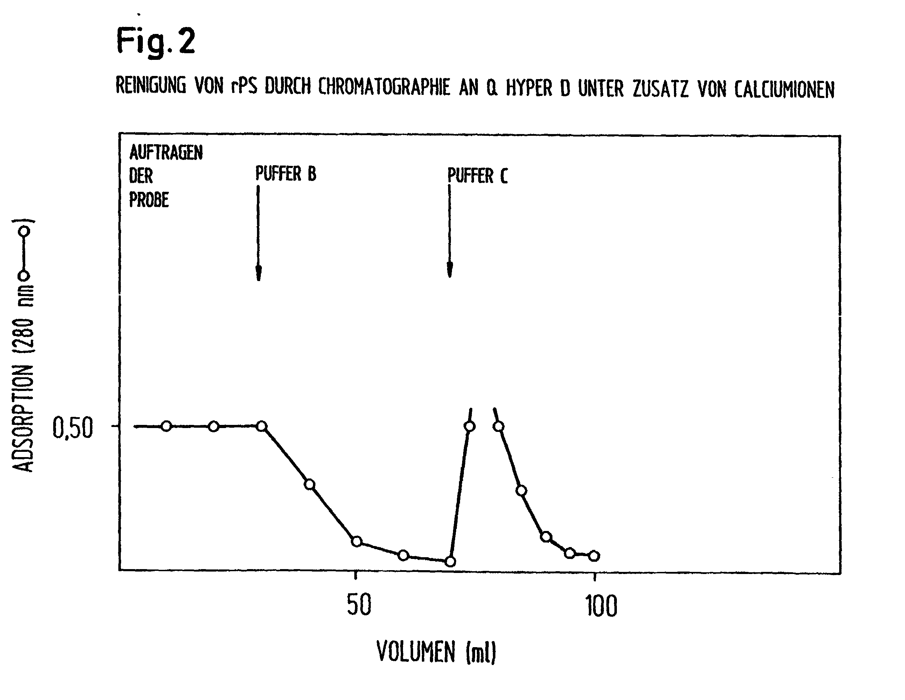

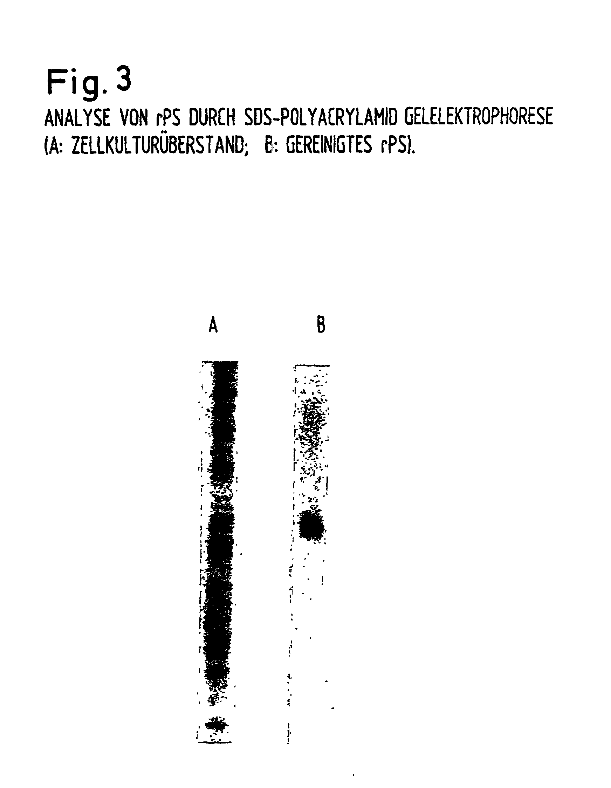

Die Ergebnisse zeigen, dass rFS die Säule ungehindert passiert, wohingegen die überwiegende Mehrzahl der weiteren Proteine an der Säule haften bleibt. Diese kontaminierenden Proteine wurden dann mit 1 M NaCl eluiert. Die wesentlichen Ergebnisse dieses Experiments sind in Fig. 2, Fig. 3 und Tabelle 2 zusammengefasst.

Durch die in Beispiel 1.b) beschriebene Reinigung wurde der Antigengehalt von rPS um das 12-fache im Verhältnis zu den anderen Proteinen erhöht. Die spezifische Aktivität erhöhte sich dabei um das 14-fache. Die denaturierende elektrophoretische Analyse (U.K. Laemmli, Nature 227, 680-685 (1970)) ergab (Fig. 3), dass durch die im Beispiel 1.b) beschriebene Reinigung rPS in mehr als 95%iger Reinheit gewonnen wurde.

**Tabelle 2**

| Probe | Volumen | Protein | Protein S | | Spezif. Aktivität |
|---|---|---|---|---|---|
| | | | Antigen | Aktivität | |
| | (ml) | (mg/ml) | (mU/ml) | (mU/ml) | (U/mg) |
| Präparation von Beispiel 1a | 34 | 0,215 | 680 | 543 | 2,5 |
| ungebundene Fraktion | 34 | 0,014 | 600 | 520 | 37 |
| 1,0 M NaCl | 10 | 0,332 | 83 | 0 | 0 |

### Beispiel 2

### a) Reinigung von rFaIX durch Anionen-Austausch-Chromatographie

Im folgenden Beispiel wurde ein quaternärer Amino-Typ Anionen-Austauscher mit Hyperdiffusionseigenschaften (Q-Hyper D, Sepracor) verwendet.

Materialien:
Säule: Q-Hyper D, Sepracor; 2 cm x 4 cm
Instrument: Pharmacia FPLC LCC-500.
Puffer A: 20 mM Bis-Tris/HCl, pH 5,5.
Puffer B: 20 mM Bis-Tris/HCl, pH 5,5, 0,18 M NaCl.
Puffer C: 20 mM Bis-Tris/HCl, pH 5,5, 0,3 M NaCl.
Puffer D: 20 mM Bis-Tris/HCl, pH 5,5, 1,0 M NaCl.

Rekombinanter Faktor IX (rFIX) wurde in analoger Weise wie für rPS (Beispiel 1.a) erhalten.

Die Säule wurde entsprechend der Vorschrift des Herstellers regeneriert und mit Puffer A equilibriert. Anschliessend wurden 1997 ml Zellkulturüberstand, welcher rekombinanten Faktor IX enthielt, mit einer Geschwindigkeit von 10 ml/min auf die Säule aufgetragen. Das nicht an die Säule gebundene Material wurde bei gleicher Fliessgeschwindigkeit durch Waschen in Puffer A entfernt. Daraufhin wurde die Säule zuerst mit Puffer B und anschliessend mit Puffer C gespült. Die Nachelution erfolgte mit Puffer D.

Während der Chromatographie wurde die ProteinAdsorption in üblicher Weise bei 280 nm verfolgt. Nach Beendigung der Chromatographie wurde die Proteinkonzentration mittels der Bradford-Methode (M Bradford, Anal. Biochem. 72, 248-254 (1976)) bestimmt. Der Gehalt an Faktor IX wurde mittels eines handelsüblichen Gerinnungstestes (Faktor IX-Gerinnung, Immuno AG) bestimmt.

Die Ergebnisse zeigen, dass nahezu der gesamte rFaIX an den Träger gebunden wurde. rFaIX wurde durch 0,3 M NaCl vom Anionen-Austauscher eluiert. Analoge Ergebnisse wurden auch bei Verwendung anderer quaternärer Amino-Typ Anionen-Austauscher erhalten. Demgegenüber wurde bei Verwendung von Q-Hyper D, der üblicherweise im Zellkulturmedium enthaltene Farbstoff Bromphenolrot bereits bei einer Salzkonzentration unterhalb 0,3 M NaCl von der Säule eluiert, was die nachfolgende Isolierung von rFaIX bei 0,3 M NaCl wesentlich begünstigte. Diese vorteilhafte Eigenschaft wiesen anderen Anionen-Austauscher nicht auf.

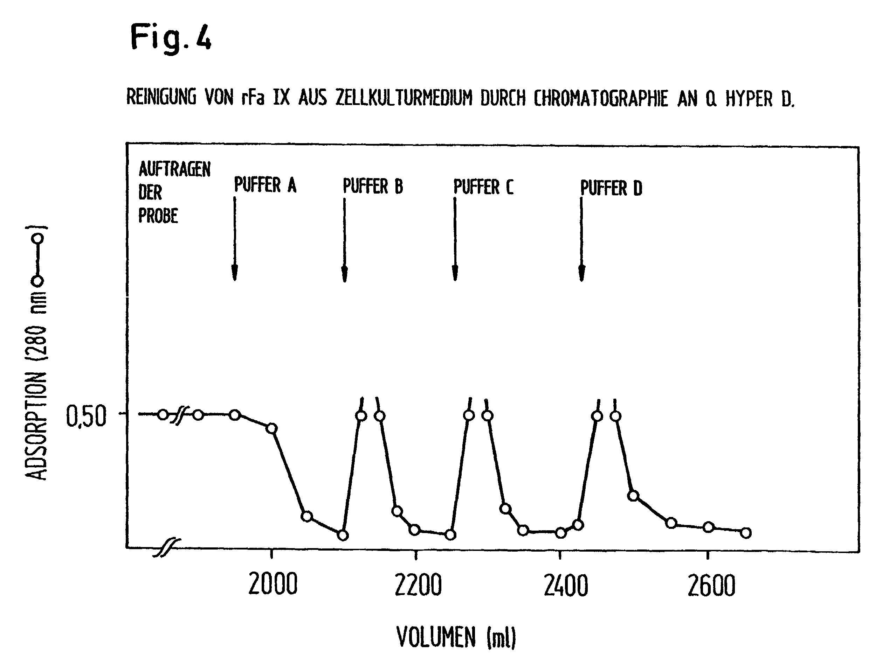

Die wesentlichen Ergebnisse der Reinigung von rFaIX am Anionen-Austauscher sind in Fig. 4 und Tabelle 3 zusammengefasst. Durch die in Beispiel 2.a) beschriebene Reinigung wurde rFaIX 20-fach angereichert; die spezifische Aktivität erhöhte sich um das 12-fache.

**Tabelle 3**

| Probe | Volumen | Protein | Faktor IX Aktivität | Spezifische Aktivität |
|---|---|---|---|---|
| | (ml) | (mg/ml) | (mU/ml) | (U/mg) |
| Zellüberstand | 1997 | 0,178 | 100 | 0,56 |
| ungebundene Fraktion | 2100 | 0,091 | 0 | 0 |
| 0,18 M NaCl | 94 | 0,382 | 162 | 0,41 |
| 0,3 M NaCl | 94 | 0,300 | 2099 | 6,86 |
| 1 M NaCl | 93 | 0,09 | 32 | 0,35 |

### b) Reinigung von rFaIX durch Adsorption von Begleitproteinen mittels Anionen-Austausch-Chromatographie unter Zusatz von Calciumionen

Als Anionen-Austauscher diente Q-Hyper D, wie er auch in Beispiel 2.a) verwendet wurde.

Materialien:
Säule: Q-Hyper D, Sepracor; 2 cm x 4 cm.
Instrument: Pharmacia FPLC LCC-500.
Puffer A: 20 mM Tris/HCl, pH 7,4.
Puffer B: 20 mM Tris/HCl, pH 7,4, 0,15 M NaCl, 10 mM CaCl₂.
Puffer C: 20 mM Tris/HCl, pH 7,4 1,0 M NaCl.

Die Säule wurde entsprechend der Vorschrift des Herstellers regeneriert und mit Puffer A equilibriert. 85 ml rekombinanter Faktor IX, wie er in Beispiel 2.a) erhalten wurde, wurden mit Puffer A 2-fach verdünnt, so dass sich die Konzentration von NaCl auf 0,15 M reduzierte. Es wurde CaCl₂ zu 10 mM zugegeben. Anschliessend wurde das Proteingemisch aufgetragen und mit Puffer B ungebundenes Protein aus der Säule gewaschen. An die Säule gebundenes Protein wurde mittels Puffer C eluiert. Der Verlauf der Chromatographie wurde wie in Beispiel 2.a) beschreiben verfolgt. Die Proteinkonzentrationen und Enzymaktivitäten wurden bestimmt.

Die Ergebnisse zeigen, dass rFaIX die Säule ungehindert passiert, wohingegen die überwiegende Mehrzahl der kontaminierenden Proteine an der Säule haften bleibt. Diese kontaminierenden Proteine wurden durch 1 M NaCl eluiert.

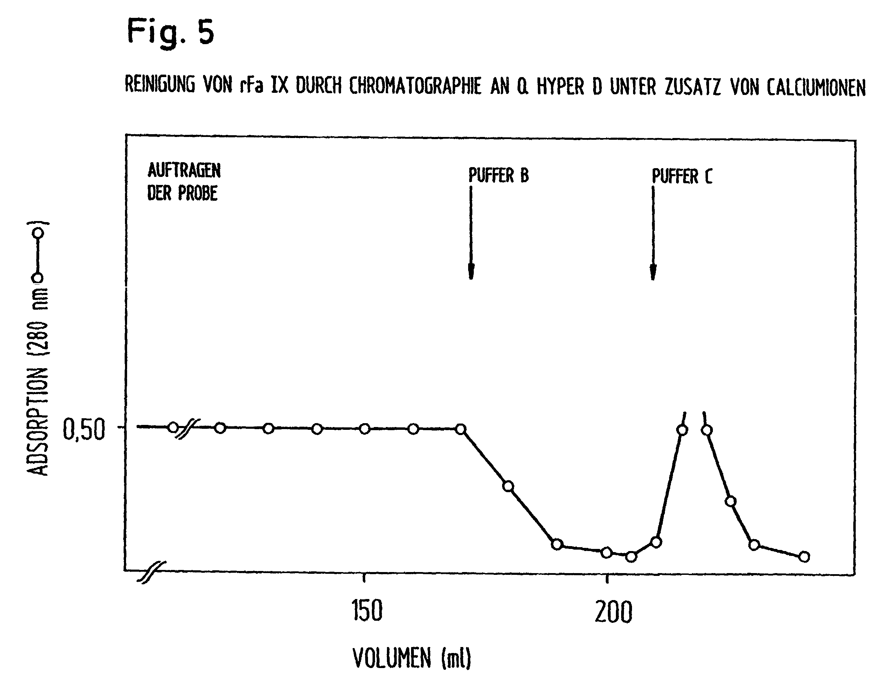

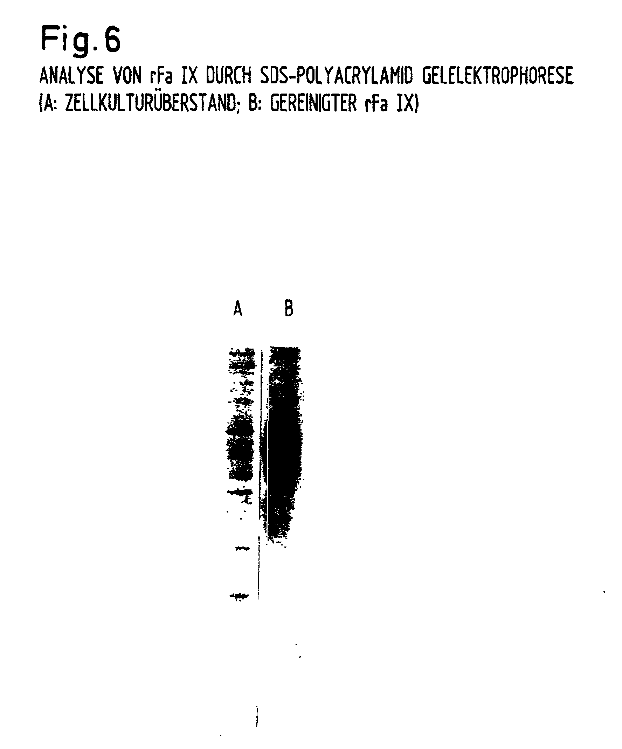

Die wesentlichen Ergebnisse sind in Fig. 5, Fig. 6 und Tabelle 4 zusammengefasst. Durch die in Beispiel 2. b) beschriebene Reinigung erhöhte sich die spezifische Aktivität von rFaIX um das 4-fache.

Die denaturierende elektrophoretische Analyse nach Laemmli ergab (Fig. 6), dass durch die in Beispiel 2.b) beschriebene Reinigung rFaIX in mehr als 80%iger Reinheit gewonnen wurde.

**Tabelle 4**

| Probe | Volumen | Protein | Faktor IX Aktivität | Spezifische Aktivität |
|---|---|---|---|---|
| | (ml) | (mg/ml) | (mU/ml) | (U/mg) |
| Präparation von Beispiel 2a | 170 | 0,153 | 1040 | 6,7 |
| ungebundene Fraktion | 200 | 0,04 | 1076 | 27,5 |
| 1 M NaCl | 20 | 0,45 | 0 | 0 |

### c) Reinigung von rFaIX durch Affinitätschromatgraphie

Im folgenden wurde rFaIX, wie in Beispiel 2.a) erhalten, durch Affinitätschromatographie gereinigt. Als Affinitätsträger wurde Heparin-Sepharose verwendet

Materialien:
Säule: HiTrap® Heparin, Pharmacia; 5 ml.
Instrument: Pharmacia FPLC LCC-500.
Puffer A: 50 mM Tris, 20 mM Natrium-Citrat, pH 7,4.
Puffer B: 50 mM Tris, 20 mM Natrium-Citrat, pH 7,4, 0,15 M NaCl.
Puffer C: 50 mM Tris, 20 mM Natrium-Citrat, pH 7,4 0,3 M NaCl.
Puffer D: 50 mM Tris, 20 mM Natrium-Citrat, pH 7,4 1 M NaCl.

Die Säule wurde entsprechend der Vorschrift des Herstellers regeneriert und mit Puffer A equilibriert. 78 ml rFaIX, wie er in Beispiel 2.a) erhalten wurde, wurde auf die Säule aufgetragen und ungebundenes Protein aus der Säule mit Puffer A ausgewaschen. Anschliessend wurde die Säule mit Puffer B, danach mit Puffer C eluiert. Die Nachelution erfolgte mit Puffer D. Der Verlauf der Chromatographie wurde wie in Beispiel 2.a) beschrieben verfolgt; die Proteinkonzentrationen und Enzymaktivitäten wurden bestimmt.

Die Ergebnisse zeigen, dass rFaIX vollständig an der Säule gebunden und erst durch 0,3 M NaCl von dieser eluiert wird. Bei diesem Verfahren werden jedoch auch weitere Proteine gleichzeitig mit rFaIX eluiert und somit nicht von diesem Faktor abgetrennt. Durch das in Beispiel 2.c) dargestellte Verfahren wurde die spezifische Aktivität von rFaIX nur um das 4,5-fache erhöht.

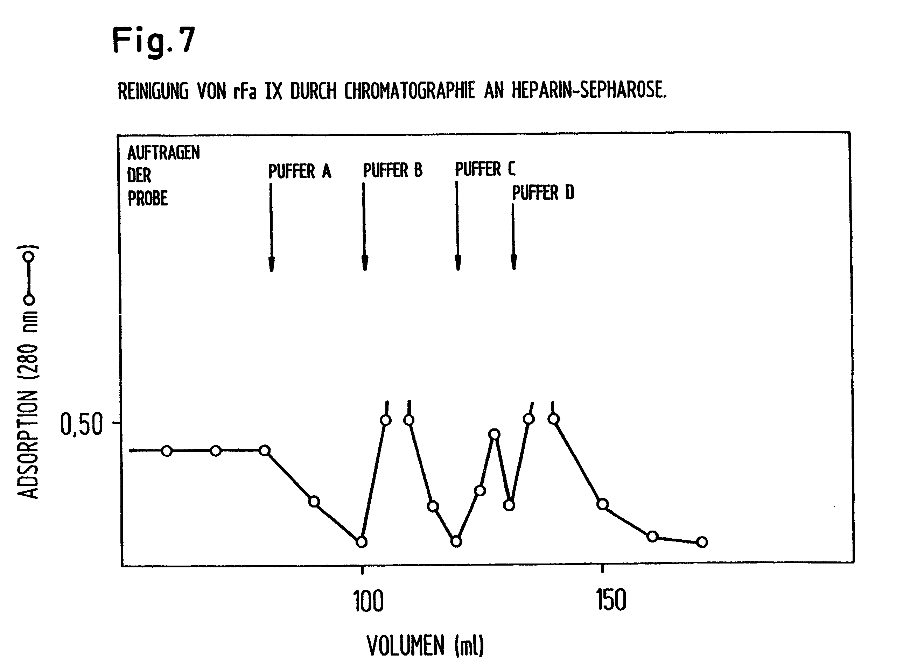

Die wesentlichen Ergebnisse sind in Fig. 7 und Tabelle 5 zusammengefasst.

**Tabelle 5**

| Probe | Volumen | Protein | Faktor IX Aktivität | Spezifische Aktivität |
|---|---|---|---|---|
| | (ml) | (mg/ml) | (mU/ml) | (U/mg) |
| Präparation von Beispiel 2a | 78 | 0,121 | 788 | 6,5 |
| ungebundene Fraktion | 90 | 0,038 | 0 | 0 |
| 0,15 M NaCl | 10 | 0,10 | 0 | 0 |
| 0,3 M NaCl | 22 | 0,053 | 1572 | 29,6 |

### Beispiel 3

### Reinigung von rFaIX durch direkte Kopplung von Anionen-Austausch-Chromatographie und Affinitäts-Chromatographie

Im folgenden Beispiel wurde rFaIX, wie im Beispiel 2.a) erhalten, dadurch gereinigt, dass dieser zuerst auf einen Anionen-Austauscher und danach direkt auf eine affinitätschromatographische Säule aufgetragen wurde. Als Anionen-Austauscher diente Q-Hyper D, dies ist ein Anionen-Austauscher vom Amino-Typ mit Hyperdiffusionseigenschaften. Als Affinitätsträger wurde Heparin-Sepharose (HiTrap® Heparin, Pharmacia) verwendet. Die Q-Hyper D-Säule war in direkter Reihung vor die Heparin-Sepharose-Säule geschlossen. Zwischen den Säulen befand sich nur eine auf das Minimum beschränkte Schlauchverbindung, gegebenenfalls mit einem Ventil.

Materialien:
Säule 1: Q-Hyper D®, Sepracor; 2 cm x 4 cm.
Säule 2: HiTrap® Heparin, Pharmacia; 5 ml.
Instrument: Pharmacia FPLC LCC-500.
Puffer A: 20 mM Tris/HCl, pH 7,4.
Puffer B: 20 mM Tris/HCl, pH 7,4, 150 mM NaCl, 10 mM CaCl₂.
Puffer C: 20 mM Tris/HCl, pH 7,4, 1,0 M MaCl.
Puffer D: 50 mM Tris, 20 mM Natrium-Citrat, pH 7,4, 0,15 M NaCl.
Puffer E: 50 mM Tris, 20 mM Natrium-Citrat, pH 7,4, 0,3 M NaCl.
Puffer F: 50 mM Tris, 20 mM Natrium-Citrat, pH 7,4, 1 M NaCl:

Der Auslauf der Säule mit dem Anionen-Austauscher wurde durch eine Schlauchverbindung direkt mit dem Einlauf der Säule der Affinitätschromatographie verbunden, so dass der Flüssigkeistsstrom zuerst durch die Säule 1 und anschliessend direkt durch die Säule 2 verlief.

Die Säulen wurden entsprechend den Vorschriften der Hersteller regeneriert und mit Puffer B equilibriert. 70 ml rFaIX, der wie in Beispiel 2.a) beschrieben aus Zellkulturüberstand isoliert worden war, wurde mit Puffer A auf das 2-fache verdünnt, so dass sich die Konzentration von NaCl auf 0,15 M reduzierte. CaCl₂ wurde bis zu einer Konzentration von 10 mM zugegeben. Anschliessend wurde das Proteingemisch bei 2,5 ml/min durch die Säule 1 (Q-Hyper D®) aufgetragen und direkt auf die Säule 2 (Heparin-Sepharose) übertragen, wobei mit Puffer B ungebundenes Protein aus den Säulen gewaschen wurde. Danach wurde die Säule 1 von der Affinitätssäule abgetrennt und der Flüssigkeitsstrom direkt auf die Säule 2 aufgetragen. In Analogie zu der in Beispiel 2.c) dargestellten Methode wurde ungebundenes Protein von der Heparin-Säule durch Waschen mit Puffer D entfernt. Die Elution der Heparin-Säule erfolgte mit Puffer E. Die Nachelution wurde mit Puffer F durchgeführt. Analoge Ergebnisse wurden erhalten, wenn nach dem Auftragen der Probe die Q-Hyper D®-Säule nicht von der Heparin-Säule entfernt, sondern der Flüssigkeitsstrom zur Elution der Heparin-Säule durch ein zwischengeschaltetes Ventil direkt auf die Heparin-Säule geleitet wurde.

Die an der Säule 1 (Q-Hyper D®) gebundenen Proteine wurden durch Puffer C eluiert.

Der Verlauf der Chromatographie wurde wie in Beispiel 2.a) beschrieben verfolgt; die Proteinkonzentration und Enzymaktivitäten wurden bestimmt.

Die Ergebnisse zeigen, dass beim Auftragen der Probe durch die vorgestellte Q-Hyper D®-Säule der rFaIX diese ungehindert passiert und vollständig auf der nachgestellten Heparin-Säule absorbiert wird. Von letzterer wird rFaIX durch 0,3 M NaCl eluiert.

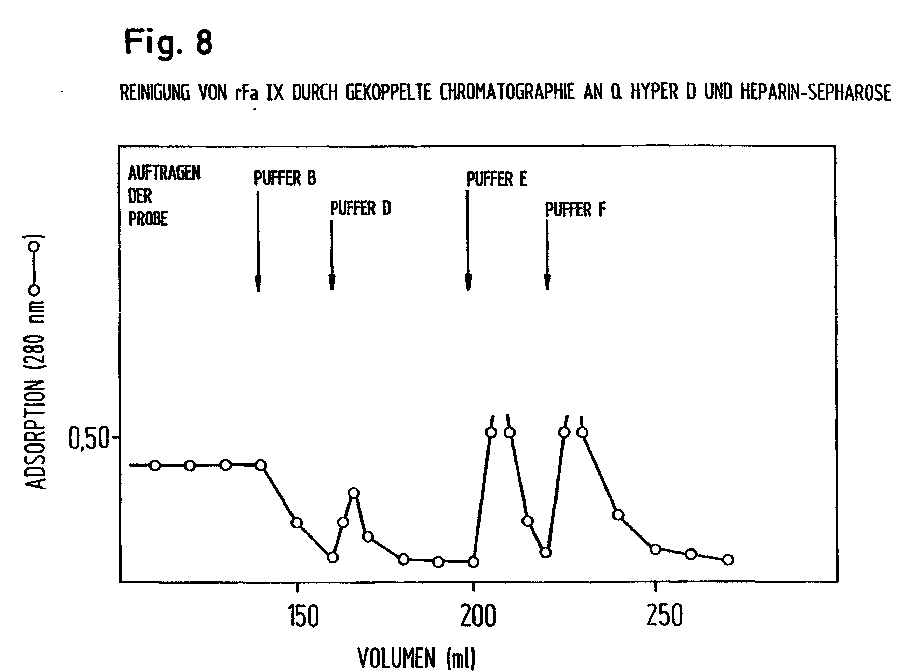

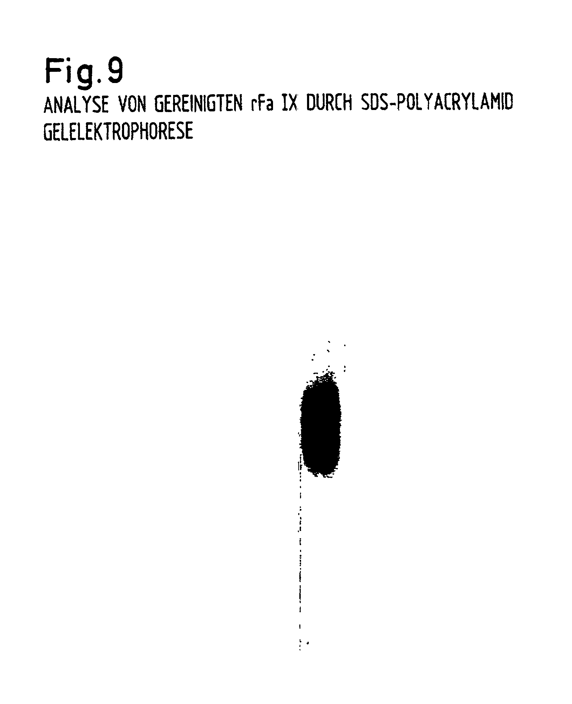

Die wesentlichen Ergebnisse sind in Fig. 8, Fig. 9 und Tabelle 6 zusammengefasst.

Durch die in Beispiel 3 beschriebene Reinigung wird rFaIX um das 8,4-fache angereichert; die spezifische Aktivität wird um das 8,8-fache erhöht. Die denaturierende elektrophoretische Analyse (nach Laemmli) ergab (Fig. 9), dass durch die im Beispiel 3 beschriebene Reinigung rFaIX in mehr als 95%iger Reinheit gewonnen wird.

Im Beispiel 3 wurde die Reinigung von rFaIX durch eine direkte Kombination der Methoden aus den Beispielen 2.a), b) und c) durchgeführt, wobei das gleiche Ausgangsmaterial eingesetzt wurde. Im Vergleich zur Reinigung aus Beispiel 2.c) wurde jedoch durch die Kombination von Anionen-Austausch-Chromatographie und Affinitätschromatographie eine höhere Anreicherung und eine höhere spezifische Aktivität erhalten. Durch die Kombination der beiden Chromatographieverfahren werden offensichtlich solche kontaminierenden Proteine, die den Trennvorgang auf dem Affinitätsträger ungünstig beeinflussen, vorweg abgetrennt, wodurch eine höhere Spezifität und Selektivität des Affinitätsträgers erreicht wird.

**Tabelle 6**

| Probe | Volumen | Protein | Faktor IX Aktivität | Spezifische Aktivität |
|---|---|---|---|---|
| | (ml) | (mg/ml) | (mU/ml) | (U/mg) |
| Präparation von Beispiel 2a | 148 | 0,0196 | 123 | 6,3 |
| ungebundene Fraktion | 160 | 0,01 | 0 | 0 |
| 0,15 M NaCl | 32 | 0,01 | 0 | 0 |
| 0,3 M NaCl | 10 | 0,019 | 1041 | 54,8 |
| 1 M NaCl | 30 | 0,015 | 0 | 0 |

### Beispiel 4

### Reinigung von rFaII durch Anionen-Austausch-Chromatographie

Im folgenden Beispiel wurde ein quaternärer Amino-Typ Anionen-Austauscher mit Tentakelgelstruktur (Fraktogel EMD TMAE 650 M, Merck) verwendet.

Materialien:
Säule: Fraktogel EMD TMAE 650 M, Merck, 1,6 cm x 5 cm Instrument: Pharmacia FPLC LCC-500 System.
Puffer A: 50 mM Tris/HCl, pH 7,4.
Puffer B: 50 mM Tris/HCl, pH 7,4, 200 mM NaCl.
Puffer C: 50 mM Tris/HCl, pH 7,4, 300 mM NaCl.
Puffer D: 50 mM Tris/HCl, pH 7,4, 1 M NaCl.

Rekombinanter Faktor II wurde, basierend auf üblichen Laborverfahren der Zellkulturtechnik gewonnen (Falkner et al, Thrombosis and Haemostatis, 68, 119-124 (1992)).

Die Expression von rFaII erfolgte in käuflich erhältlichem, synthetischem DMEM-Medium. Zellfreier Kulturüberstand wurde durch Zentrifugation erhalten

Die Säule wurde entsprechend der Vorschrift des Herstellers regeneriert und mit Puffer A equilibriert. Anschliessend wurden 200 ml Zellkulturüberstand, welcher rekombinanten Faktor II enthielt, mit einer Geschwindigkeit von 4 ml/min auf die Säule aufgetragen. Nicht an die Säule gebundenes Material wurde bei gleicher Fliessgeschwindigkeit durch Waschen mit Puffer A entfernt. Im folgenden wurde die Säule zuerst mit Puffer B und anschliessend mit Puffer C gespült. Dann wurde mit Puffer D nacheluiert. Während der Chromatographie wurde die ProteinAdsorption in üblicher Weise bei 280 nm verfolgt. Nach der Chromatographie wurde die Proteinkonzentation mittels der Bradford-Methode bestimmt. Der Gehalt an Faktor II wurde sowohl mit Hilfe eines handelsüblichen ELISA-Systems (Asserachrome Protein S, Boehringer Mannheim) als auch mit Hilfe eines Gerinnungstestes (Thrombinzeit, Immuno AG) bestimmt.

Es wurde gefunden, dass nahezu der gesamte rFaII an den Träger gebunden wurde. rFaII wurde durch 0,3 M NaCl vom Anionen-Austauscher eluiert. Ausserdem wurde - im Gegensatz zu anderen Amino-Typ Anionen-Austauschern, wie z.B. MacroPrep® (Bio-Rad) oder Q-Speharose Fast Flow (Pharmacia) - der üblicherweise im Zellkulturüberstand enthaltene Farbstoff Bromphenolrot bereits bei einer Salzkonzentration von 0,2 M NaCl von der Säule eluiert, was die nachfolgende Isolierung von rFaII bei 0,3 M NaCl wesentlich begünstigte.

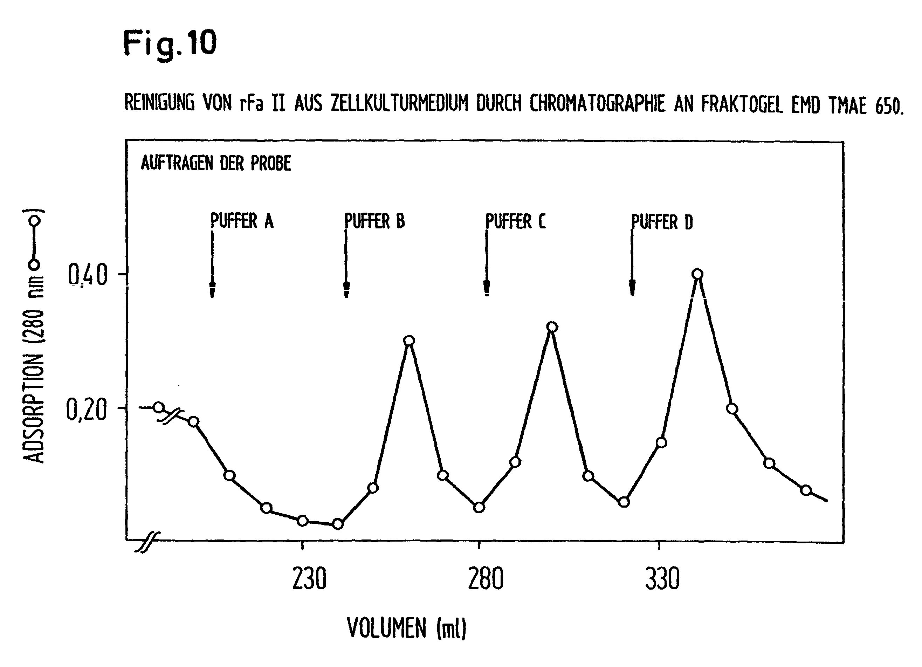

Die wesentlichen Ergebnisse der Reinigung von rPS am Anionen-Austauscher sind in Fig. 10 und Tabelle 7 zusammengefasst. Durch die im Beispiel 4 beschriebene Reinigung erhöhte sich die spezifische Aktivität von rFaII und das 3,5-fache.

**Tabelle 7**

| Probe | Volumen | Protein | Faktor II Aktivität | Spezifische Aktivität |
|---|---|---|---|---|
| | (ml) | (mg/ml) | (mU/ml) | (U/mg) |
| Zellmedium | 200 | 0,45 | 50 | 0,1 |
| ungebundene Fraktion | 200 | 0,177 | 2 | 0,01 |
| 0,2 M NaCl | 40 | 0,4 | 6 | 0,015 |
| 0,3 M NaCl | 45 | 0,36 | 150 | 0,42 |
| 0,5 M NaCl | 32 | 0,25 | 0 | 0 |

### b) Reinigung von rFaII durch Adsorption von Begleitproteinen mittels Anionen-Austausch-Chromatographie unter Zusatz von Calciumionen

Als Anionen-Austauschharz diente Fraktogel EMD TMAE 650 M, Merck).

Materialien:
Säule: Fraktogel EMD TMAE 650 M, 1,6 cm x 5 cm.
Instrument: Pharmacia FPLC LCC-500.
Puffer A: 50 mM Tris/HCl, pH 7,4.
Puffer B: 50 mM Tris/HCl, pH 7,4, 150 mM NaCl, 10 mM CaCl₂.
Puffer C: 50 mM Tris/HCl, pH 7,4, 1,0 mM NaCl.

Die Säule wurde entsprechend der Vorschrift des Hersteller regeneriert und mit Puffer B equilibiert. Rekombinanter Faktor II, welcher wie in Beispiel 4 a) beschrieben, aus Zellkulturüberstand isoliert worden war, wurde mit Puffer A 2-fach verdünnt, so dass die Konzentration von NaCl weniger als 0,2 M betrug. Es wurde CaCl₂ bis zu einer Konzentration von 10 mM zugegeben. Anschliessend wurde das Proteingemisch auf die Säule aufgetragen und mit Puffer B ungebundenes Protein aus der Säule gewaschen. Gebundenes Protein wurde mittels Puffer C eluiert.

Der Verlauf der Chromatographie wurde wie in dem vorangehenden Beispiel verfolgt und die Proteinkonzentrationen bestimmt. Die Ergebnisse zeigen, dass rFaII die Säule ungehindert passiert, wohingegen die überwiegende Mehrzahl der kontaminierenden Proteine an der Säule haften bleibt. Diese Proteine wurden dann durch 1 M NaCl eluiert.

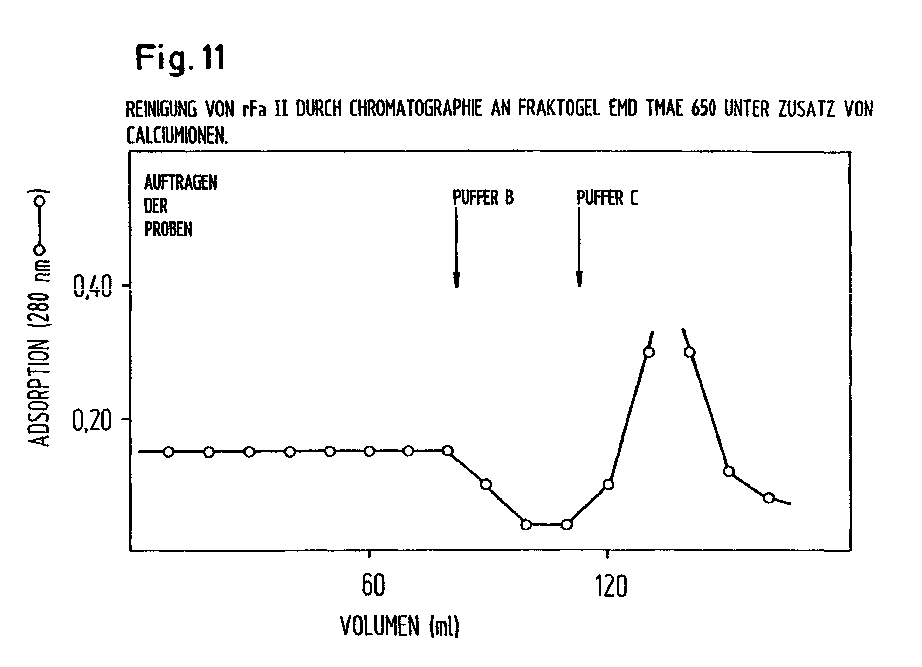

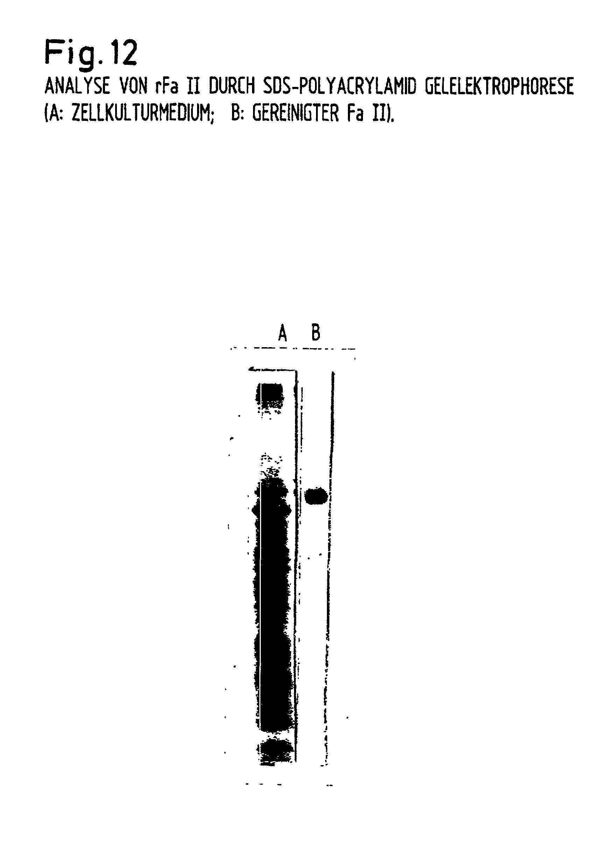

Die wesentlichen Ergebnisse sind in Fig. 11, Fig. 12 und Tabelle 8 zusammengefasst. Durch die in Beispiel 4.a) und b) beschriebene Reinigung wurde die spezifische Aktivität von rFaII um das 11-fache erhöht.

Die denaturierende elektrophoretische Analyse nach Laemmli ergab (Fig. 9), dass durch die im Beispiel 4a und b beschriebene Reinigung rFa II in mehr als 95 %-iger Reinheit gewonnen wurde.

**Tabelle 8**

| Probe | Volumen | Protein | Faktor II Aktivität | Spezifische Aktivität |
|---|---|---|---|---|
| | (ml) | (mg/ml) | (mU/ml) | (U/mg) |
| Präparation von Beispiel 4a | 80 | 0,18 | 75 | 0,41 |
| ungebundene Fraktion | 80 | 0,015 | 75 | 4,6 |
| 1,0 M NaCl | 15 | 0,62 | 0 | 0 |

## Patentansprüche

1. Verfahren zur Trennung eines Vitamin K-abhängigen Proteins von nicht-Vitamin K-abhängigen Begleitproteinen aus einer proteinhaltigen Lösung, **dadurch gekennzeichnet**, dass zu dieser Lösung Calciumionen zugegeben werden und die calciumhaltige Lösung mit einem Anionenaustauscher unter Bedingungen in Kontakt gebracht wird, bei denen die Begleitproteine, jedoch nicht das Vitamin K-abhängige Protein, adsorbiert werden.

2. Verfahren nach Anspruch 1, **dadurch gekennzeichnet**, dass die das Vitamin K-abhängige Protein enthaltende Lösung zusätzlich mit einem Anionenaustauscher unter Bedingungen in Kontakt gebracht wird, bei denen das Vitamin K-abhängige Protein an den Austauscher adsorbiert, gewaschen, eluiert und gegebenenfalls die Ionenstärke des Eluats reduziert wird.

3. Verfahren nach Anspruch 1, **dadurch gekennzeichnet**, dass das nicht-adsorbierte Vitamin K-abhängige Protein an einen Affinitätsträger adsorbiert und selektiv eluiert wird.

4. Verfahren nach Anspruch 1, **dadurch gekennzeichnet**, dass es mit einem Verfahrensschritt nach Anspruch 2 und/oder 3 in beliebiger Kombination und Reihenfolge durchgeführt wird.

5. Verfahren nach Anspruch 1 und 2, **dadurch gekennzeichnet**, dass die Trennschritte mit dem gleichen Anionenaustauscher durchgeführt werden, vorzugsweise mit Hilfe von Chromatographie.

6. Verfahren nach einem der Ansprüche 1 bis 5, **dadurch gekennzeichnet**, dass der Anionenaustauscher Hyperdiffusionseigenschaften besitzt.

7. Verfahren nach Anspruch 6, **dadurch gekennzeichnet**, dass als Anionenaustauscher der Träger Q-Hyper D® verwendet wird.

8. Verfahren nach einem der Ansprüche 1 bis 5, **dadurch gekennzeichnet**, dass der Anionenaustauscher Tentakelstruktur besitzt.

9. Verfahren nach Anspruch 3, **dadurch gekennzeichnet**, dass die lonenstärke der Proteinlösung nach der Elution auf 0,15 bis 0,2 M herabgesetzt wird.

10. Verfahren nach Anspruch 1 bis 9, **dadurch gekennzeichnet**, dass das Vitamin K-abhängige Protein ausgewählt ist aus FII, FVII, FIX, FX, Protein S, Protein C und Protein Z.

11. Verfahren zur Gewinnung eines Vitamin K-abhängigen Proteins gemäß einem der Ansprüche 1 bis 10.

12. Verfahren zur Gewinnung eines nicht-Vitamin K-abhängigen Begleitproteins gemäß einem der Ansprüche 1 bis 10.

## Claims

1. Process for separating a vitamin K-dependent protein from non-vitamin K-dependent concomitant proteins from a protein-containing solution, **characterised in that** calcium ions are added to this solution and the calcium-containing solution is contacted with an anion exchanger under conditions in which the concomitant proteins, but not the vitamin K-dependent protein, are absorbed.

2. Process according to claim 1, **characterised in that** the solution containing vitamin K-dependent protein is additionally contacted with an anion exchanger under conditions in which the vitamin K-dependent protein is adsorbed on the exchanger, washed, eluted and optionally the ionic strength of the eluate is reduced.

3. Process according to claim 1, **characterised in that** the non-adsorbed vitamin K-dependent protein is adsorbed on an affinity carrier and selectively eluted.

4. Process according to claim 1, **characterised in that** it is carried out using a process step according to claim 2 and/or 3 in any combination and sequence.

5. Process according to claim 1 and 2, **characterised in that** the separating steps are carried out using the same anion exchanger, preferably with the aid of chromatography.

6. Process according to one of claims 1 to 5, **characterised in that** the anion exchanger has hyperdiffusion properties.

7. Process according to claim 6, **characterised in that** the carrier Q-Hyper D® is used as anion exchanger.

8. Process according to one of claims 1 to 5, **characterised in that** the anion exchanger has tentacle structure.

9. Process according to claim 3, **characterised in that** the ionic strength of the protein solution is reduced to 0.15 to 0.2 M after elution.

10. Process according to claim I to 9, **characterised in that** the vitamin K-dependent protein is selected from FII, FVII, FIX, FX, protein S, protein C and protein Z.

11. Process for recovering a vitamin K-dependent protein according to one of claims 1 to 10.

12. Process for recovering a non-vitamin K-dependent concomitant protein according to one of claims 1 to 10.

## Revendications

1. Procédé de séparation d'une protéine vitamine K dépendante depuis des protéines d'accompagnement non vitamine K dépendante, à partir d'une solution contenant des protéines, **caractérisé en ce que** l'on ajoute à cette solution des ions calcium et la solution contenant du calcium étant mise en contact avec un échangeur d'anions, dans des conditions pour lesquelles les protéines accompagnatrices sont adsorbées, mais cependant pas la protéine vitamine K dépendante.

2. Procédé selon la revendication 1, **caractérisé en ce que** la solution contenant la protéine vitamine K dépendante est en plus mise en contact avec un échangeur d'anions, dans des conditions dans lesquelles la protéine vitamine K dépendante est adsorbée, lavée, éluée sur l'échangeur et, le cas échéant, la force ionique de l'éluat étant réduite.

3. Procédé selon la revendication 1, **caractérisé en ce que** la protéine vitamine K dépendante n'ayant pas été adsorbée est adsorbée sur un support à affinités et éluée de façon sélective.

4. Procédé selon la revendication 1, **caractérisé en ce qu**'il est mis en oeuvre avec une étape de procédé selon la revendication 2 et/ou 3, en une combinaison et un ordre de succession quelconques.

5. Procédé selon les revendications 1 et 2, **caractérisé en ce que** les étapes de séparation sont effectuées avec le même échangeur d'anions, de préférence à l'aide d'une chromatographie.

6. Procédé selon les revendications 1 à 5, **caractérisé en ce que** l'échangeur d'anions possède des propriétés d'hyperdiffusion.

7. Procédé selon la revendication 6, **caractérisé en ce qu**'on utilise comme échangeur d'anions le support Q-Hyper D® .

8. Procédé selon les revendications 1 à 5, **caractérisé en ce que** l'échangeur d'anions possède une structure tentaculaire.

9. Procédé selon la revendication 3, **caractérisé en ce que** la force ionique de la solution de protéine, après élution est abaissée à une valeur comprise dans la plage de 0,15 à 0,2 M.

10. Procédé selon les revendications 1 à 9, **caractérisé en ce que** la protéine vitamine K dépendante est sélectionnée parmi FII, FVII, FIX, FX, protéine S, protéine C et protéine Z.

11. Procédé d'obtention d'une protéine vitamine K dépendante, selon l'une des revendications 1 à 10.

12. Procédé d'obtention d'une protéine accompagnatrice, non vitamine K dépendante, selon l'une des revendications 1 à 10.
